# FASCICULE DE BREVET EUROPEEN

(11) **EP 1 529 500 B1**
(45) Date de publication et mention de la délivrance du brevet: **03.01.2007**
(21) Numéro de dépôt: 03078517.4
(22) Date de dépôt: 07.11.2003
(51) Int. Cl.: A61F 2/36

(54) **Dispositif pour remplacer les articulations osseuses de la hanche**
Vorrichtung zum Ersatz des Hüftgelenks
Device for replacing the hip joint articulation

(43) Date de publication de la demande: 11.05.2005
(73) Titulaire: Bone and Joint Research S.A., 3650 Kayl (LU)
(72) Inventeur: Autrique, Jean Claude, Dr., 1300 Wavre (BE); Bogaert, Johan, Dr., 3540 Herk-de-Stad (BE); Boucquey, Pierre, Dr., 7700 Mouscron (BE); Deneufbourg, Jean, Dr., 1300 Wavre (BE); Geulette, Bernard,Dr., 1640 Rhodes St Genese (BE); Ghijselings, Ignace, Dr., 9910 Ursel (BE); Ghosez, Jean-Pierre, Dr., 5081 Bovesse (BE); Labrique, Jean-François, Dr., 7711 Dottignies (BE); Legaye, Jean, Dr., 5340 Gesves (BE); Manche, Eric, Dr., 7063 Soignies (BE); Oosterlinck, Dirk, Dr., 8792 Waregem (BE); Poilvache, Pascal, Prof., 1060 Bruxelles (BE); Thiéry, Jacques, Dr., 1390 Gresz-Doiceau (BE); Vander Maren, Christian, Dr., 1340 Ottignies (BE); Goube, Michel, 62152 Hardelot (FR); Bigand, Dominique, 62600 Groffliers (FR); Thiltgès, Paul, 1020 Bruxelles (BE); Thiltgès, Laurence, 1020 Bruxelles (BE)
(74) Mandataire: Waxweiler, Jean

(56) Documents cités:
- WO-A-01/05337
- FR-A- 2 639 820
- FR-A- 2 784 576
- US-B1- 6 436 147

## Description

La présente invention concerne un dispositif pour le remplacement des articulations osseuses de la hanche selon la partie précaractérisante de la revendication 1.

Les prothèses fémorales sont d'usage courant et sont implantées dans l'extrémité supérieure du fémur pour remplacer la tête fémorale, en cas notamment de fracture ou d'arthrose sévère.

Ainsi qu'il est bien connu le fémur et essentiellement sa région trochantéro-céphalique subit des contraintes mécaniques, et l'axe mécanique de cette région correspond à la face postérieure de l'os. Ces contraintes mécaniques sont sous la dépendance des puissants muscles péri-articulaires et de la pesanteur avec des différences notables selon la position du membre inférieur et au cours des différentes phases de la marche:
a)Au repos en station allongée: la contraction statique des muscles psoas iliaque, grand et moyen fessiers qui forment un couple autour de l'axe longitudinal du fémur constitue la seule source de contrainte.
b)Au cours de la marche il apparaît des forces de pression et d'inflexion internes du fémur pour l'équilibre de la hanche en appui bi- et unipodal.

Ces notions ont été mieux cernées grâce aux travaux de PAUWELS: ainsi parle t-on des colonnes de PAUWELS ou de la balance de PAUWELS.

La théorie de PAUWELS est basée sur la transmission des forces à une colonne vertébrale selon que la charge est appliquée dans l'axe de la colonne ou avec un bras de levier. La charge appliquée dans l'axe vertical de la colonne (axe neutre) détermine des contraintes de pression verticale, par contre appliquée avec un bras de levier les contraintes observées sont des contraintes de pression et des contraintes de flexion latérale (forces d'inflexion, voire de cisaillement) au niveau de la colonne. Cette théorie de la balance de Pauwels est bien connue de ces spécialistes et ne nécessite pas d'explications plus détaillées ici. On se référera par exemple à la publication de Pauwels dans Z. Orthop. Ihre Grenzgeb. 1975 Feb.; 113 (1) : 1-5.

Les dispositifs connus à ce jour pour remplacer les articulations du membre inférieur de type sphérique permettant de corriger la position relative entre l'axe de la cavité recevant le piton d'ancrage de la prothèse et la position du centre de rotation idéal de l'articulation sphérique sont de trois types:
1. La publication FR-A-2619005 décrit des implants dits «homothétiques» dont le col reliant le piton d'ancrage et la tête ou sphère articulaire présente une longueur proportionnelle à la grosseur du piton d'ancrage. Ce principe corrélant la longueur du col à la grosseur du piton d'ancrage ne permet pas de régler correctement la tension des muscles fessiers en cas de morphologie atypique du membre ou lorsque le canal médullaire de l'os est étroit ou très large. En effet le choix chirurgicale du piton est en relation avec ladite cavité médullaire, donc, en cas de canal très large, par exemple sur un patient âgé et de morphologie moyenne, l'opérateur doit implanter un piton de gros calibre, donc par homothétie avec un col trop long qui va créer une tension excessive sur les muscles fessiers, source de douleurs et de boiterie.
2. La publication FR-A-2666737 décrit des implants dit «varisés ou valgisés» dont le col reliant le piton d'ancrage et la sphère articulaire présente une longueur constante mais est plus ou moins incliné par rapport à l'axe du piton d'ancrage. (Voir aussi la publication FR-A-2631543). Par sa conception, un col valgisé (ou verticalisé), s'il permet effectivement de tendre les muscles fessiers, rapproche le membre opéré de l'axe médian du corps tout en l'allongeant ce qui oblige souvent le port d'un talon de semelle corrective sur le membre non opéré d'une hauteur compensant l'allongement chirurgical. A l'inverse un col varisé (ou horizontalisé) raccourcit légèrement le membre tout en l'écartant de l'axe médian du corps; le col ainsi abaissé risque également de venir précocement en contact avec le bord de la coquille d'ancrage osseux en position assise.
3. La publication FR-A-2638350 décrit des implants dits «médialisés ou latéralisés» dont le col reliant le piton d'ancrage et la sphère articulaire présente un angle et une longueur constante mais est translaté pour éloigner plus ou moins l'axe du piton d'ancrage du centre de rotation et donc rapproche ou écarte le membre de l'axe médian du corps. Ce principe présente l'avantage de ne pas allonger ni raccourcir le membre, mais crée un déséquilibre de face en appui bipodal, avec parfois un frottement des genoux dû à la médialisation, ou encore une rotation externe du membre par hypertension des muscles fessiers en cas de latéralisation excessive.

Ces différents types d'implants bien que séduisants par leurs principes respectifs sont assez décevants à moyen terme car ils ne tiennent pas compte du changement d'inclinaison dans le plan frontal du bassin selon la position d'appui lors de la marche, pouvant conduire à un conflit entre le col de la prothèse fémorale et le bord de la cupule avec pour conséquence une luxation ou un descellement nécessitant le remplacement des implants.

Il est nécessaire d'offrir aux patients des implants pour le remplacement des articulations de hanches, indolores, stables, exempts de luxation même en cas de grande mobilité, afin de réduire les remplacements de prothèses sur une population âgée, pour laquelle les interventions de reprise sont lourdes de conséquences.

La présente invention est définie par la revendication 1. L'objet de l'invention est obtenu d'une part entre le col de ladite prothèse qui est lisse pour permettre le frottement doux exempt d'usure, et entre le chanfrein situé à l'ouverture de la cupule, et cette ouverture se rétrécit vers le centre de ladite cupule articulaire, et l'angle du chanfrein est conçu pour s'adapter parfaitement avec le col de la prothèse, dans les positions extrêmes des pièces lorsque le col vient en contact avec le bord évasé de la cupule, en cas de débattement de grande amplitude - et d'autre part par le réglage précis de la position relative du centre de l'articulation sphérique et de l'axe du piton d'ancrage afin de respecter l'iso-tension des muscles fessiers lors des changements de l'appui mono-podal/bi-podal caractéristiques des phases de marche pour réaliser un équilibre musculo-ligamentaire proche de la balance de PAUWELS naturelle. Ainsi qu'on l'a dit plus haut cet équilibre est bien connu des spécialistes de la biomécanique qui, ont montré que la posture change par bascule latérale et rotation des ailes iliaques autour des têtes fémorales anatomiques (ou prothétiques).

Ce pivotement de l'ensemble du bassin avec ses insertions musculaires se répercute sur la colonne vertébrale et les membres inférieurs en y induisant des torsions, en particulier lors de la montée ou de la descente d'escaliers.

Lors de ce mécanisme de bascule-rotation du bassin en position debout, le col de la prothèse vient précocement en contact avec le bord de la cupule avec un risque accru d'usure, de luxation et/ou de descellement de ladite cupule.

D'autre part en cas de modification ou de non respect des bras de levier décrits dans la balance de Pauwels, par exemple si une prothèse médialisée ou de col trop court est mise en place, le patient doit compenser par une augmentation de l'effort des muscles fessiers - ceci devient critique pour les patients âgés et/ou à faible musculature.

Il s'agit donc d'un mécanisme auquel les systèmes décrits ci-dessus n'apportent aucune réponse face aux problèmes fonctionnels tels que l'amplitude réduite de mouvements, la luxation ou le descellement, pour les articulations de la hanche.

Les caractéristiques les plus importantes de l'invention permettant de corriger ces défauts sont présentées dans la description et réitérées dans les revendications.

Le dispositif de remplacement des articulations osseuses de la hanche est défini dans la partie caractérisante de la revendication 1.

D'autres avantages et aspects de l'invention ressortiront à la lecture de la description suivante faite à titre d'exemple et en référence aux dessins annexés dans lesquels:

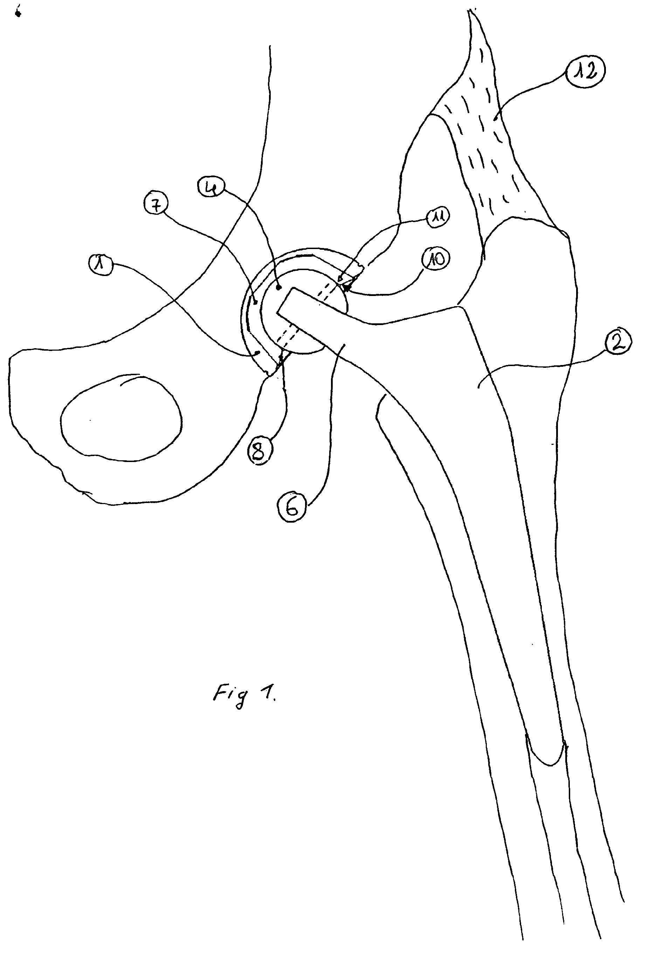

La figure 1 est une vue en perspective du dispositif selon l'invention comportant un système d'articulation osseuse constitué de trois composants articulaires, à savoir une coquille d'ancrage osseux et sa cupule articulaire glissant sur une tête ou bille fixée au moyen du col sur une tige ou piton d'ancrage d'une prothèse fémorale de hanche.

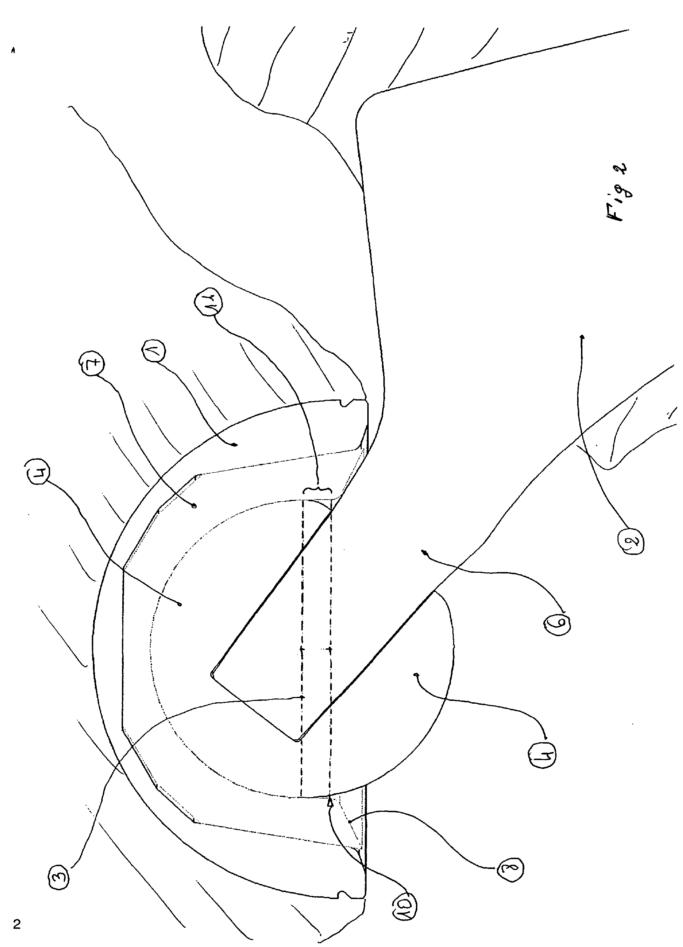

La figure 2 est une vue plus en détail montrant une position articulaire extrême avec les caractéristiques revendiquées pour le col et pour l'évasement interne de l'ouverture de la cupule articulaire.

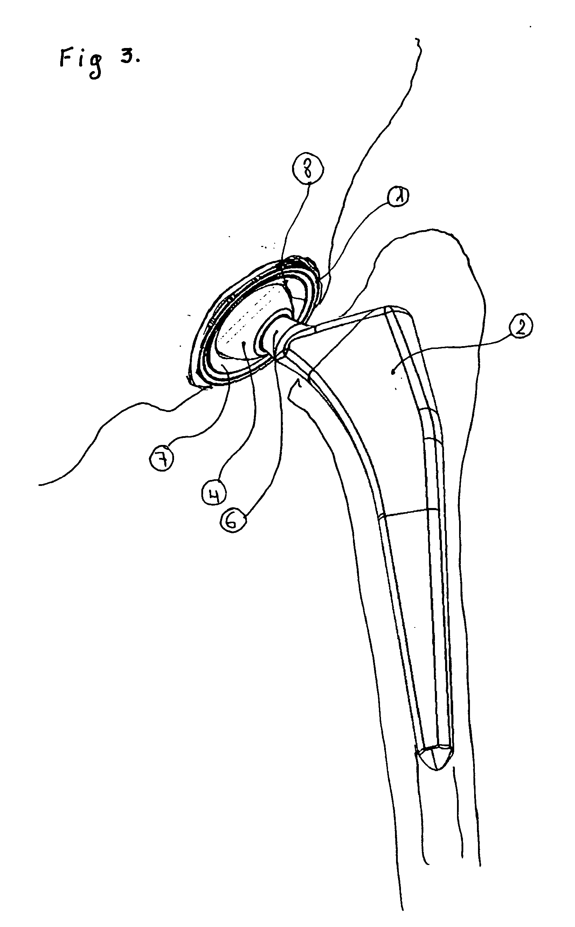

La figure 3 est un schéma décrivant les principes d'iso-tension que l'on cherche à restaurer en fonction de la balance de PAUWELS, celle-ci étant calculée en relation avec la largeur du bassin.
Les figures 4 et 5 montrent à titre d'exemples les différents modèles de cols 6 en relation avec la taille du piton d'ancrage 2 dans chaque famille, permettant de réaliser l'iso-tension des muscles fessiers, tout en évitant un conflit avec ledit évasement interne de l'ouverture 8 de la cupule articulaire 7.

L'invention n'est pas limitée aux formes de réalisation représentées dans les dessins annexés qui ont un but purement illustratif.

Le dispositif ou prothèse pour remplacer les articulations osseuses de la hanche est constitué d'un ensemble de pièces réalisées en matériaux bio-compatibles et comprend une tige ou piton d'ancrage 2 prolongé par un col 6, de préférence évasé depuis son extrémité extérieure vers le piton d'ancrage. Ce col reçoit une tête ou bille 4 polie, laquelle rapporte une cupule articulaire sensiblement hémisphérique qui vient se loger dans une coquille d'ancrage 1 osseux se fixant dans le bassin.

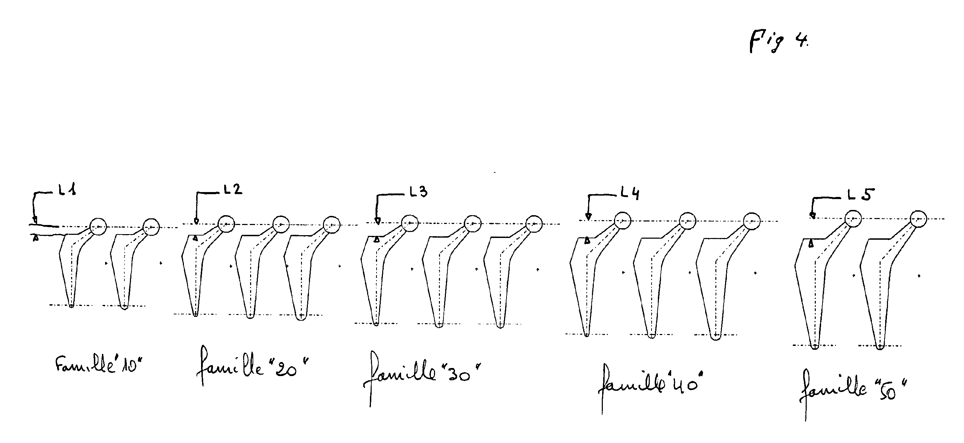

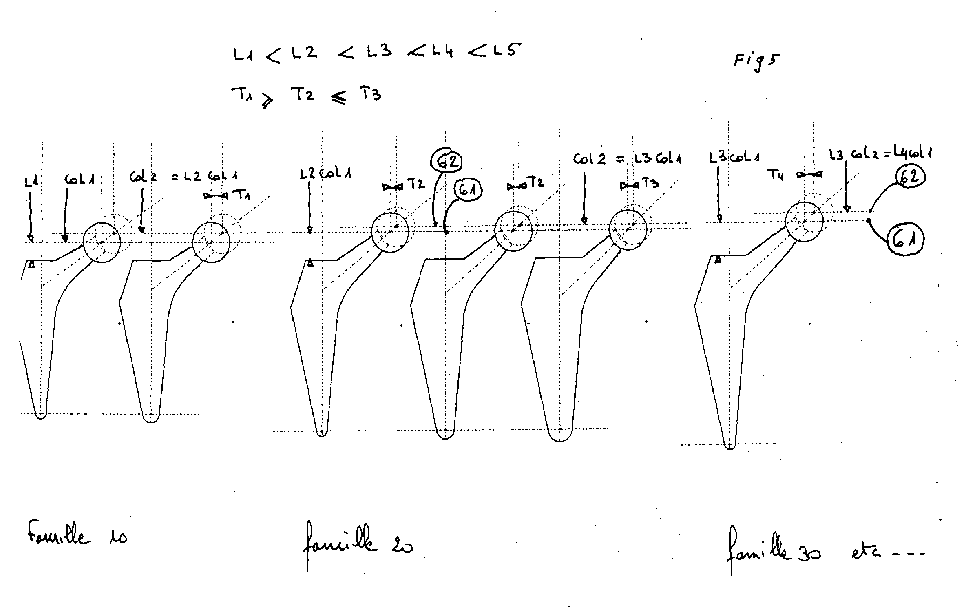

Le dispositif selon l'invention se caractérise en ce qu'il comprend au moins trois composants avec deux surfaces de contact et de glissement concourant au déplacement.
- La coquille d'ancrage osseux 1 est en matériau rigide et se caractérise en ce que le début du bord évasé de son ouverture 10 est situé à une distance comprise entre 0.5 et 3mm au-delà de l'équateur 3 de la cupule articulaire 7 de manière à réaliser un guidage cylindrique dont l'évasement est conçu pour s'adapter parfaitement avec le col 6 évasé de la prothèse de hanche, dans toutes les positions respectives des pièces.
- La partie du piton d'ancrage 2 opposée au piton d'ancrage reçoit la bille 4 polie et le col 6 qui est caractérisé en ce qu'il est lisse de préférence légèrement évasé, pour correspondre à l'évasement interne de l'ouverture 8 de la cupule articulaire 7 pour permettre un contact exempt d'usure.
- Une tête ou bille 4 polie fixée sur le col 6 du piton d'ancrage 2 par un moyen d'assemblage de préférence conique.
Ce col 6 est décliné ou présenté sous au moins deux dimensions pour chaque taille du piton d'ancrage 2 de manière à permettre de choisir la tension idéale des muscles fessiers 12 tout en restaurant le déport de la diaphyse fémorale pour respecter la balance de Pauwels: la longueur du col 6 augmente de préférence de façon non homothétique pour chaque famille 10 à 50 de tailles du piton d'ancrage 2 (voir les fig. 4 et 5) et est déclinée pour chaque famille 10 à 50 en au moins deux longueurs 61 et 62 telles que la plus longue d'une famille 10 à 50 corresponde à la plus courte de la famille 10 à 50 de piton d'ancrage 2 de taille immédiatement supérieure. A titre d'exemple dans la figure 5, la longueur du petit col L3 de la famille 30 est égale à celle du col long L2 de la famille 20 et le col long L3 de la famille 30 est égal au col court L4 de la famille 40.

Du fait de la distribution gaussienne (c-à-d selon la courbe de Gauss) des tailles de fémur chez les patients, le nombre de pitons d'ancrage 2 est de préférence variable dans chaque famille 10 à 50.

Ce système d'articulation permet grâce à ses caractéristiques particulières d'assurer avec succès, même en cas de révision ou de mauvaise qualité osseuse, un choix précis de la position de la diaphyse tant en translation horizontale qu'en allongement du membre et donc un réglage équilibré de la tension des muscles fessiers 12 entre l'appui mono-podal et bi-podal, pour obtenir un remplacement stable et durable permettant des mouvements de grande amplitude sans luxation ni usure pour les articulations de hanche.

Le dispositif pour le remplacement des articulations osseuses de la hanche se caractérise également en ce que la dimension du col 6 de référence dans chaque famille de piton d'ancrage 2 est en relation avec le sommet du grand trochanter tant en hauteur, qu'en translation latérale afin de respecter l'équilibre de la balance de Pauwels en appui mono-podal.

En outre, la dimension en hauteur du col 6 de référence dans une famille de piton d'ancrage 2 est déterminée par une ligne théorique passant par le sommet des grands trochanters des deux fémurs afin de respecter l'équilibre global du bassin en appui bi-podal.

Bien que l'invention a été décrite et illustrée en rapport avec un mode de réalisation particulier, il est bien entendu qu'elle n'est pas limitée à celui-ci et qu'elle doit être considérée dans le cadre des revendications annexées.

## Revendications

1. Dispositif de remplacement des articulations de hanche, en matériaux bio-compatibles, comprenant au moins trois composants articulaires avec deux surfaces de contact de glissement concourant aux déplacements, à savoir:
- une coquille d'ancrage osseux (1) en matériau rigide recevant à sa face interne concave une cupule articulaire lisse (7) sensiblement hémisphérique polie;
- une prothèse (5) comportant un piton d'ancrage (2) ayant une longueur et à la partie opposée de ce piton d'ancrage diaphysaire (2) un col (6) lisse, pour permettre un frottement exempt d'usure et de préférence légèrement évasé, le col ayant une dimension (61,62) prédéterminée;
- une tête ou bille (4) polie fixée sur le col (6) du piton d'ancrage (2) par un moyen d'assemblage de préférence conique connu en soi;
**caractérisé en ce qu**'il comporte pour chaque longueur du piton d'ancrage (2) au moins deux familles (10 à 50) ayant chacune au moins deux tailles de piton d'ancrage et au moins deux dimensions (61, 62) de col (6) différentes telles que la plus longue dimension de col d'une famille (10 à 50) corresponde à la plus courte dimension de col de la famille (10 à 50) de piton d'ancrage (2) de longueur immédiatement supérieure, afin de permettre un choix précis de la position de la diaphyse tant en translation horizontale qu'en allongement du membre et donc un réglage équilibré de la tension des muscles fessiers (12) entre l'appui mono-podal et bi-podal.

2. Dispositif selon la revendication 1, **caractérisée en ce que** le début du bord évasé de l'ouverture (10) de cette cupule articulaire lisse (7) est situé à une distance comprise entre de 0.5 et 3 mm au-delà de l'équateur (3) de cette cupule articulaire de manière à réaliser un guidage (11) de préférence cylindrique et son évasement est conçu pour s'adapter parfaitement ensemble avec le col (6) évasé de la prothèse (5), dans toutes les positions respectives des pièces.

3. Dispositif selon la revendication 1 **caractérisé en ce que** ce col (6) est **caractérisé par** une progression non homothétique de sa dimension entre chaque famille (10 à 50) .

4. Dispositif selon l'une quelconque des revendications 1 à 3 dont le piton d'ancrage (6) est **caractérisé en ce qu**'il est décliné en familles de taille, selon la distribution gaussienne des tailles des fémurs chez les patients, chaque famille comprenant une dimension de col (6) dite de référence, propre à ladite famille.

5. Dispositif selon l'une quelconque des revendications 1 à 4 **caractérisé en ce que** le piton d'ancrage (2) est décliné pour chaque famille (10 à 50) en au moins deux dimensions de col (6) de manière à permettre de choisir la tension idéale des muscles fessiers (12) tout en restaurant le déport de la diaphyse fémorale pour respecter la balance de Pauwels.

6. Dispositif selon la revendication 5 **caractérisé en ce que** le nombre de pitons d'ancrage (2) est de préférence variable dans chaque famille (10 à 50) en tenant compte de la distribution selon la courbe de Gauss des tailles de fémur des patients.

7. Dispositif selon l'une des revendications 1 à 5 **caractérisé en ce que** la dimension du col (6) de référence dans chaque famille de piton d'ancrage (2) est en relation avec le sommet du grand trochanter (13) tant en hauteur, qu'en translation latérale afin de respecter la longueur des bras de leviers déterminant l'équilibre de la balance de Pauwels en appui mono-podal.

8. Dispositif selon la revendication 7 **caractérisé en ce que** la dimension en hauteur du col (6) de référence dans une famille de piton d'ancrage (2) est déterminée par une ligne théorique (14) passant par le sommet des grands trochanter (13) de deux fémurs afin de respecter l'équilibre global du bassin en appui bi-podal.

9. Dispositif selon la revendication 7 **caractérisé en ce que** la dimension en translation latérale du col (6) de référence dans la famille de piton d'ancrage (2) est déterminée par une symétrie selon le plan sagittal du corps, la position étant déterminée pour la balance de PAUWELS sur le membre opposé afin de respecter l'équilibre musculaire du bassin en appui mono-podal.

10. Dispositif selon l'une quelconque des revendications 1 à 9 **caractérisé en ce qu**e la longueur du col (6) augmente de préférence de façon non homothétique pour chaque famille (10 à 50) de tailles du piton d'ancrage (2) et est déclinée pour chaque famille (10 à 50) en au moins deux longueurs (61 et 62) telles que la plus longue d'une famille (10 à 50) corresponde à la plus courte de la famille (10 à 50) de piton d'ancrage (2) de taille immédiatement supérieure.

11. Dispositif selon la revendication 10 **caractérisé en ce que** la longueur du petit col (L3) d'une famille (30) est égale à celle du col long (L2) de la famille (20) de piton d'ancrage (2) de longueur immédiatement inférieure et le col long (L3) de la même famille (30) est égal au col court (L4) de la famille (40) de piton d'ancrage (2) de longueur immédiatement supérieure.

## Claims

1. A hip joint replacement device, in biocompatible materials, comprising at least three articular components with two sliding contact surfaces working together to produce displacements, that is:
- a bone anchoring housing (1) in a rigid material receiving at its inner concave face a smooth articular cup (7) that is substantially hemispherical and polished;
- a prosthesis (5) comprising an anchoring element (2) having a length, and at the part opposite from this shaft anchoring element (2), a smooth collar (6) that is preferably slightly flared to allow wear-free friction, the collar having a predetermined dimension (61, 62);
- a polished head or ball fixed on the collar (6) of the anchoring element (2) by an assembling means that is preferably conical and known in itself;
**characterized in that** the device comprises, for each length of the anchoring element (2), at least two families (10 to 50), each having at least two sizes of anchoring elements and at least two dimensions (61, 62) of different collars (6) such that the longest collar dimension of a family (10 to 50) corresponds to the shortest collar dimension of the family (10 to 50) of the anchoring element (2) with the next longer length, allowing the position of the shaft in horizontal translation and extension of the member and therefore a balanced adjustment of the tension of the gluteus muscles (12) between monopodal and bipodal weightbearing to be precisely selected.

2. The device according to claim 1 **characterized in that** the start of the flared edge of the opening (10) of this smooth articular cup (7) is situated at a distance between 0.5 and 3 mm beyond the equator (3) of this articular cup in such a way as to produce a guide (11), preferably cylindrical, and its flare is designed to perfectly fit together with the flared collar (6) of the prosthesis (5), in all respective positions of the pieces.

3. The device according to claim 1 **characterized in that** this collar (6) is **characterized by** a non-homothetic progression of its dimension between each family (10 to 50) .

4. The device according to any one of claims 1 to 3 wherein the anchoring element (6) is **characterized in that** the element is available in size families, according to the Gaussian distribution of femur sizes in patients, each family comprising a collar dimension (6) called the reference dimension, specific to said family.

5. The device according to any one of claims 1 to 4 **characterized in that** the anchoring element (2) is available for each family (10 to 50) in at least two collar dimensions (6) in such a way as to allow the ideal tension of the gluteus muscles (12) to be selected while restoring the misalignment of the femoral shaft to respect Pauwels balance.

6. The device according to claim 5 **characterized in that** the number of anchoring elements (2) is preferably variable in each family (10 to 50) by taking the distribution according to the Gaussian curve of the femur sizes of patients into account.

7. The device according to one of claims 1 to 5 **characterized in that** the reference collar (6) dimension in each anchoring element (2) family is in relation with the end of the greater trochanter (13) in height and lateral translation to respect the length of the lever arms determining the equilibrium of Pauwels balance in monopodal weightbearing.

8. The device according to claim 7 **characterized in that** the height dimension of the reference collar (6) in an anchoring element (2) family is determined by a theoretical line (14) passing by the end of the greater trochanters (13) of both femurs to respect the overall equilibrium of the pelvis in bipodal weightbearing.

9. The device according to claim 7 **characterized in that** the dimension in lateral translation of the reference collar (6) in the anchoring element family (2) is determined by a symmetry according to the sagittal plane of the body, the position being determined for Pauwels balance on the opposite member to respect the muscular equilibrium of the pelvis in monopodal weightbearing.

10. The device according to any one of claims 1 to 9 **characterized in that** the length of the collar (6) preferably increases in a non-homothetic manner for each family (10 to 50) of anchoring element (2) sizes and is available for each family (10 to 50) in at least two lengths (61 and 62) such that the longest of one family (10 to 50) corresponds to the shortest of the family (10 to 50) of the anchoring element (2) in the next longer size.

11. The device according to claim 10 **characterized in that** the length of the small collar (L3) of a family (30) is equal to that of the long collar (L2) of the family (20) of the anchoring element (2) with the next shorter length and the long collar (L3) of the same family (30) is equal to the short collar (L4) of the family (40) of the anchoring element (2) with the next longer length.

## Patentansprüche

1. Vorrichtung zum Ersetzen von Hüftgelenken aus biokompatiblen Werkstoffen, die mindestens drei Gelenkbauteile mit zwei Gleitberührungskontaktflächen aufweist, die bei den Bewegungen beitragen, nämlich:
- eine Knochenverankerungsschale (1) aus starrem Werkstoff, die auf ihrer konkaven Innenseite eine glatte, im Wesentlichen halbkugelförmiger polierte Gelenkcupula (7) aufnimmt,
- eine Prothese (5), die einen Verankerungsstift (2) mit einer Länge aufweist und an dem entgegen gesetzten Teil dieses diaphysären Verankerungsstifts (2) einen glatten Kragen (6), um ein Reiben ohne Abnutzung zu erlauben, und der vorzugsweise leicht aufgeweitet ist, wobei der Kragen ein vorbestimmtes Maß (61, 62) hat,
- einen Kopf oder eine polierte Kugel (4), die auf dem Kragen (6) des Verankerungsstifts (2) durch ein vorzugsweise als solches bekanntes kegeliges Zusammenfügemittel befestigt ist,
**dadurch gekennzeichnet, dass** sie für jede Länge des Verankerungsstifts (2) mindestens zwei Familien (10 bis 50) aufweist, die jeweils mindestens zwei Größen von Verankerungsstiften haben und mindestens zwei unterschiedliche Maße (61, 62) des Kragens (6), so dass das längere Kragenmaß einer Familie (10 bis 50) dem kürzeren Kragenmaß der Familie (10 bis 50) von Verankerungsstiften (2) mit gleich darüber liegender größerer Länge entspricht, um eine genaue Auswahl der Diaphysenposition sowohl in horizontaler Verschiebung als auch in Streckung des Glieds zu erlauben und daher eine ausgewogene Einstellung der Spannung der Gesäßmuskeln (12) zwischen der monopodalen und bipodalen Auflage.

2. Vorrichtung nach Anspruch 1, **dadurch gekennzeichnet, dass** der Beginn des aufgeweiteten Rands der Öffnung (10) dieser glatten Gelenkcupula (7) in einer Entfernung zwischen 0,5 et 3 mm über dem Äquator (3) dieser Gelenkcupula liegt, um eine vorzugsweise zylindrische Führung (11) herzustellen, und dass seine Aufweitung so konzipiert ist, dass sie sich perfekt an den aufgeweiteten Kragen (6) der Prothese (5) in allen jeweiligen Positionen der Teile anpasst.

3. Vorrichtung nach Anspruch 1, **dadurch gekennzeichnet, dass** dieser Kragen (6) durch einen nicht homothetischen Verlauf seines Maßes zwischen jeder Familie (10 bis 50) **gekennzeichnet** ist.

4. Vorrichtung nach einem der Ansprüche 1 bis 3, bei der der Verankerungsstift (6) **dadurch gekennzeichnet ist, dass** er in Größenfamilien gemäß der Gauß'schen Verteilung der Oberschenkelknochen bei den Patienten existiert, wobei jede Familie ein Kragenmaß (6) aufweist, das Referenzmaß genannt wird, das der Familie eigen ist.

5. Vorrichtung nach einem der Ansprüche 1 bis 4, **dadurch gekennzeichnet, dass** der Verankerungsstift (2) für jede Familie (10 bis 50) aus mindestens zwei Kragenmaßen (6) besteht, so das es erlaubt wird, die ideale Spannung der Gesäßmuskeln (12) auszuwählen und gleichzeitig den Versatz der Oberschenkeldiaphyse wiederherzustellen, um das Pauwels-Gleichgewicht einzuhalten.

6. Vorrichtung nach Anspruch 5, **dadurch gekennzeichnet, dass** die Anzahl der Verankerungsstifte (2) vorzugsweise in jeder Familie (10 bis 50) variabel ist, wobei die Verteilung gemäß der Gauß'schen Kurve der Oberschenkelgrößen der Patienten berücksichtigt wird.

7. Vorrichtung nach einem der Ansprüche 1 bis 5, **dadurch gekennzeichnet, dass** das Referenzkragenmaß (6) jeder Familie von Verankerungsstiften (2) eine Beziehung zu dem Gipfel des Trochanter major (13) sowohl in der Höhe als auch in der seitlichen Verschiebung hat, um die Länge der Hebelarme einzuhalten, die das Gleichgewicht des Pauwels-Gleichgewichts bei monopodalem Aufliegen bestimmen.

8. Vorrichtung nach Anspruch 7, **dadurch gekennzeichnet, dass** das Höhenmaß des Referenzkragens (6) in einer Familie von Verankerungsstiften (2) von einer theoretischen Linie (14) bestimmt ist, die durch den Gipfel der Trochanter major (13) von zwei Oberschenkelknochen verläuft, um das globale Gleichgewicht des Beckens bei bipodaler Auflage einzuhalten.

9. Vorrichtung nach Anspruch 7, **dadurch gekennzeichnet, dass** das Seitenverschiebungsmaß des Referenzkragens (6) in der Verankerungsstiftfamilie (2) von einer Symmetrie entlang der sagittalen Ebene des Körpers bestimmt ist, wobei die Position für das Pauwels-Gleichgewicht auf dem entgegen gesetzten Glied bestimmt ist, um das Muskelgleichgewicht des Beckens beim monopodalen Aufliegen einzuhalten.

10. Vorrichtung nach einem der Ansprüche 1 bis 9, **dadurch gekennzeichnet, dass** die Länge des Kragens (6) vorzugsweise nicht homothetisch für jede Familie (10 bis 50) von Größen des Verankerungsstifts (2) steigt und für jede Familie (10 bis 50) in mindestens zwei Längen (61, 62) existiert, so dass die längere einer Familie (10 bis 50) der kürzeren der Familie (10 bis 50) von Verankerungsstiften (2) mit unmittelbar darüber liegender Größe entspricht.

11. Vorrichtung nach Anspruch 10, **dadurch gekennzeichnet, dass** die Länge des kleinen Kragens (L3) einer Familie (30) gleich der des langen Kragens (L2) der Familie (20) von Verankerungsstiften (2) mit unmittelbar kleinerer Länge ist, und der lange Kragen (L3) der gleichen Familie (30) gleich dem kurzen Kragen (L4) der Familie (40) von Verankerungsstiften (2) mit gleich darüber liegende größerer Länge ist.
